(19) Europäisches Patentamt
European Patent Office
Office européen des brevets

(11) **EP 3 031 455 B1**

(12) **EUROPEAN PATENT SPECIFICATION**

(45) Date of publication and mention
of the grant of the patent:
**22.11.2017 Bulletin 2017/47**

(51) Int Cl.:
*A61K 31/198* (2006.01)          *B82Y 5/00* (2011.01)
*A61P 25/20* (2006.01)

(21) Application number: **15197931.7**

(22) Date of filing: **04.12.2015**

(54) **AGENT FOR THE TREATMENT AND PREVENTION OF SLEEP DISORDERS**

MITTEL ZUR BEHANDLUNG UND VORBEUGUNG VON SCHLAFSTÖRUNGEN

AGENT POUR LE TRAITEMENT ET LA PRÉVENTION DE TROUBLES DU SOMMEIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.12.2014 RU 2014149306**

(43) Date of publication of application:
**15.06.2016 Bulletin 2016/24**

(73) Proprietor: **ZAO "ALMAZ PHARM"
117393 Moscow (RU)**

(72) Inventors:
• **LEONIDOV, Nikolay
117036 Moscow (RU)**
• **VORONINA, Tatiana
119048 Moscow (RU)**
• **YAKOVLEV, Ruslan
300001 Tula (RU)**

(74) Representative: **Jeck, Anton
Jeck & Fleck
Patentanwälte
Klingengasse 2
71665 Vaihingen/Enz (DE)**

(56) References cited:
**US-A1- 2014 162 066**

• **MAKOTO BANNAI ET AL: "New Therapeutic
Strategy for Amino Acid Medicine: Glycine
Improves the Quality of Sleep", JOURNAL OF
PHARMACOLOGICAL SCIENCES, vol. 118, no. 2,
1 January 2012 (2012-01-01), pages 145-148,
XP055250072, JP ISSN: 1347-8613, DOI:
10.1254/jphs.11R04FM**
• **DATABASE WPI Week 201425 Thomson
Scientific, London, GB; AN 2014-C81681
XP002754286, -& RU 2 506 075 C1 (LEONIDOV N
B) 10 February 2014 (2014-02-10)**
• **DATABASE WPI Week 201581 Thomson
Scientific, London, GB; AN 2015-64642F
XP002754287, -& RU 2 566 713 C1 (LEONIDOV N
B) 27 October 2015 (2015-10-27)**

**Description**

The present invention relates to medicine, in particular, to psychopharmacology, and involves the use of the known sedative: glycine immobilized on detonation nanodiamond particles 2-10 nm in size [1] (hereinafter referred to as Almacin), for the treatment and prevention of sleep disorders regardless of the cause of said disorders.

Sleep is a specific physiological state of the body, predominantly the brain, which is vitally important for higher animals and humans and thus, a sharp rise in the number of individuals suffering from sleep disorders is a serious medical and social problem. Sleep disorder is presently one of the most commonly diagnosed disorders of the central nervous system. Various sleep disorders affect more than one quarter of the entire world population [2].

Sleep disorders affect both healthy individuals of all ages at the time of various difficult situations, and the individuals afflicted with a wide range of medical conditions.

Sleep disorders accompany psychiatric (chronic stress, neurosis, depression, schizophrenia, etc.) and neurological (stroke, headache, Alzheimer's disease, Parkinson's disease, epilepsy, migraine, multiple sclerosis, vertebrobasilar insufficiency, etc.) conditions, various pain syndromes (arthritis, osteoarthritis, osteochondrosis, trauma, cancer, etc.), hormonal changes (pregnancy, menopause, aging, hyperthyroidism, etc.), renal failure, bronchial asthma, chronic and acute cardiac failure, hypertension and some other conditions.

According to different classifications, there are 70 sleep disorder syndromes with characteristic changes in the amount and quality of sleep, sleep times, behavior, and various sleep-related physiological conditions. The most common symptom is insomnia, which is characterized by a subjective sensation of dissatisfaction with sleep, not being rested after sleep with overall decrease in sleep duration, difficulty falling asleep, and frequent waking up. Insomnia sharply alters the structure of sleep, which may be expressed in the decreased duration of both slow-wave and fast-wave sleep phases, and in the reduction of the occurrences thereof [2].

There are two approaches to the treatment of sleep disorders [2]. The first is nonspecific and applies behavioral modification methods and psychotherapy. The second approach is directed to the treatment of specific nosological forms of insomnia. It comprises affecting the causative factors of sleep disorder and/or direct impact on its structure with different pharmaceutical substances. The first stage of modern pharmacotherapy irrefutably started in 1903, when barbiturates were first used for the treatment of insomnia. The second stage began in the 1950s, when neuroleptics (mostly phenothiazine derivatives) and antihistamines were first used. The third stage was the stage of benzodiazepines, which began in 1960 with chlordiazepoxide; diazepam was launched in 1963, and oxazepam - in 1965. The forth stage began in the 1980s, with the development and use of cyclopyrrolones, imidazopyridines, and later pyrazolopyrimidines.

In Russia, the most common drugs used for the treatment of sleep disorders are benzodiazepines, which are strong sedative-hypnotics (phenazepam, nitrazepam, diazepam, etc.). They act as receptors with a wide therapeutic range and relatively low toxicity. That said, the preparations of this group cause significant problems to the patients (increasing as the dose increases), such as: addiction, dependence, withdrawal syndrome, increased sleep apnea, memory loss, anterograde amnesia, attention deficit, decreased response time, daytime drowsiness. No benzodiazepines are recommended for the treatment of chronic insomnia because of the danger of drug dependence and addiction.

Most sedative-hypnotic drugs inhibit the postsynaptic GABAergic complex. They include barbiturates, benzodiazepines, cyclopyrrolones, imidazopyridines, and pyrazolopyrimidines. Other medications used for treating insomnia are neuroleptics having a sedative effect, antidepressants having sedative and chronobiotic effects, as well as antihistamines. Preparations based on all these pharmacological groups specifically and nonspecifically affect the sleep structure. Nonspecific effects on sleep structure are: increased sleep time, shortened sleep onset latency, shortened sleep stages 1 and 2 and lengthened deep sleep stages of slow-wave sleep, wakefulness duration and movement during sleep. Specific effects of the preparations are tied to the effect on specific neurotransmitter systems. For instance, most nonselective GABAergic preparations prolong stage 2 of slow-wave sleep and shorten REM stages. Tricyclic antidepressants suppress REM.

Sedative effect of the nonbenzodiazepines is associated with the non-GABAergic system modulation of the brain. At the present time, the most commonly used sleep disorder treatment and prevention agents of that type are antihistamines, which are central histamine $H_1$-receptor antagonists. One of the most widely used sedative-hypnotic drugs of this type is Donormyl (doxylamine), which lengthen the periods and quality of sleep while not changing the sleep phase. It has not been associated with any signs of addiction or withdrawal. The medication is safe for pregnant women during the entire course of pregnancy under a physician's supervision. Patients treated with said medication reported significant improvements in the time to sleep onset, prolonged sleep duration, reduced wakefulness at night, and better quality of morning wakefulness, and their total sleepiness scale score reached levels almost comparable to those of healthy individuals. Polysomnographic study results showed reduced time to sleep onset, prolonged sleep duration, extended REM, and an improved sleep quality index [2]. However, sedative-hypnotic drugs available today do not fully satisfy clinical demands since these symptomatic agents are not adequately effective and cause substantial side effects, such as tolerance and a need to increase the dose, as well as causing drug dependence (and first and foremost this refers to benzodiazepines) [2].

Finding novel sedative-hypnotic drugs, therefore, is a pressing, socially significant and global-scale issue.

The nonessential amino acid glycine ($NH_2CH_2COOH$), which is a central inhibitory neurotransmitter, is known to exhibit sedative effects and improve the metabolic processes in brain tissue [3,4]. Glycine is currently used in medical practice as an agent reducing the urge for alcohol consumption, tempering withdrawal symptoms, moderating depression and high irritability, normalizing sleep, and also in combination therapy for the treatment of cerebral circulation disorders [3, 4]. However, because of its weak sedative-hypnotic effect, glycine is not used as a sedative-hypnotic agent in medical practice.

The pharmacological basis of glycine is founded on the amplification effect of the metabolic and neurotransmitter processes triggered by the enhancement of its endogenic synthesis. Intracellular synthesis of glycine can only be increased with signal pathways mediated by the interaction with receptor systems. Interaction of glycine with the receptors thereof opens chloride channels, hyperpolarizes the membrane, and amplifies the inhibitory effect. Furthermore, glycine can act as an allosteric co-agonist of glutamate receptors. Binding in a specific site, it enhances glutamate's and И N-methyl-D-aspartate's (NMDA) ability to open cation channels [4,5].

A glycinergic system as well as GABA were found to play a significant role in sleep regulation. Glycinergic neurons were shown to inhibit somatic motor neurons during REM sleep, and glycine reduces the time periods of wakefulness and overall duration of wakefulness [6]. The sedative mechanism of glycine is associated with its ability to inhibit the activity of orexin neurons, which are affected by orexins, the endogenous hypothalamic peptides, which regulate the sleep-wake cycles (wakefulness and REM stages) [7].

A conjugate of glycine immobilized on detonation nanodiamond particles 2-10 nm in size (hereinafter referred to as Almacin) exhibiting sedative, antidepressant, antipsychotic, and anxiolytic activity greatly exceeding the corresponding types of specific activities of the pharmacopeia glycine, and a method for producing thereof are known in the art [1,8,9,10]. The antidepressant effect of Almacin is at least as great as that of the reference antidepressants amitriptyline and fluoxetine. Furthermore, in doses exceeding the recommended therapeutic dose of glycine 20-fold, it did not cause any side or toxic effects.

No pronounced sedative-hypnotic effect of the conjugate of glycine immobilized on detonation nanodiamond particles (Almacin) was reported in the patent or scientific literature, neither was the application thereof for the treatment and prevention of sleep disorders.

The present invention describes an agent for the treatment and prevention of sleep disorders, which is a conjugate of glycine immobilized on detonation nanodiamond particles 2-10 nm in size containing up to $21 \pm 3$ wt.% of glycine.

To determine whether the conjugate of glycine immobilized on detonation nanodiamond particles 2-10 nm in size (Almacin) could be used in medicine as an agent for the treatment and prevention of sleep disorders, the sedative-hypnotic activity thereof was compared to that of the pharmacopeia glycine and the known sedative-hypnotic pharmaceutical preparation Donormyl (comparator agent).

The study of the sedative-hypnotic effect of Almacin vs. comparator agents was conducted on 35 mature outbred male white rats weighing 230-250 g. The study was conducted in accordance with "Methodological Guidelines for Conducting a Study of Sedative-Hypnotic Activity of Pharmaceutical Substances" as set forth in "The Guidance Manual for Experimental (non-clinical) Studies of Novel Pharmaceutical Substances" [11].

The neurophysiological (electrophysiological) criteria of various stages of the sleep-wake cycle were analyzed. Electrodes were surgically implanted into the sensorimotor area of the cerebral cortex, dorsal hippocampus, and neck muscles of Nembutal-anesthetized rats (50 mg/kg, intramuscular) in an encephalometer, in accordance with the brain atlas, 5 - 7 days prior to the experiments on sleep monitoring. Electrobiological activity of the brain (electroencephalography, EEG) was recorded for unrestrained animals in the morning/daytime hours for at least 5 hrs. Sleep phases were determined experimentally by the electrical activity of the cerebral cortex and dorsal hippocampus, and by the electromyogram. In the analysis of sleep-wake cycles, the software recorded the following indicators: latent time from the beginning of registration to the appearance of the first episode of slow-wave and fast-wave (paradoxical, REM) sleep; total number of slow-wave and fast-wave sleep episodes and episodes of wakefulness; duration of all slow-wave and fast-wave sleep episodes and all episodes of wakefulness; percentage of all slow-wave and fast-wave sleep episodes and all episodes of wakefulness calculated by the ratio between the duration of said indicators and the duration of the total EEG record.

The agent of the present invention Almacin was shown to enhance the theta-rhythm synchronization and modulation in the electrograms of the sensorimotor cortex and dorsal hippocampus in the fast-wave (paradoxical) sleep stage, which indicates the normalizing effect of the substance on cortical-diencephalic interactions involved in the electrophysiological and neurochemical balance regulations in the central nervous system during sleep. Almacin demonstrated a clear sedative-hypnotic effect, which was confirmed by a statistically significant decrease in sleep onset latency: first episodes of slow-wave and fast-wave (paradoxical, REM) sleep; increased duration and percentage of fast-wave sleep and decreased total number of the duration and percentage of the wakefulness phase episodes.

The pharmacological study confirmed that the agent of the present invention for the treatment and prevention of sleep disorders exhibits a specific and pronounced sedative-hypnotic effect more potent than the sedative-hypnotic effect of the pharmacopeia preparation glycine or Donormyl. That gave way to the creation of a greater variety of effective and safe sedative-hypnotic medications.

Brief description of the drawings.

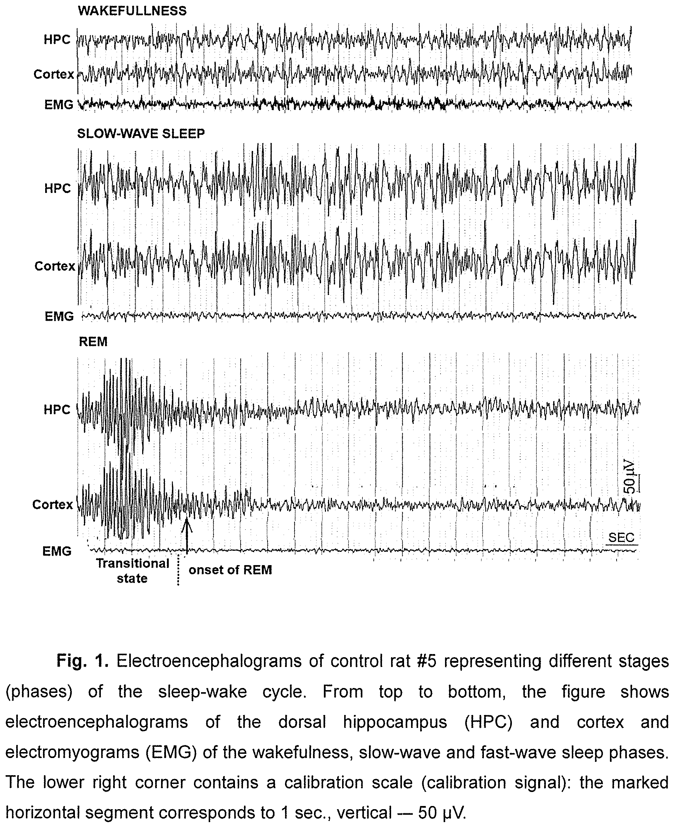

Fig.1. Electroencephalograms of control rat #5 showing different stages (phases) of the sleep-wake cycle. From top to bottom the figure shows electroencephalograms of the dorsal hippocampus (HPC) and cortex (cortex) and electromyograms (EMG) of the wakefulness, slow-wave, and fast-wave sleep phases. The lower right corner contains a calibration scale (calibration signal): the marked horizontal segment corresponds to 1 sec., vertical - 50 $\mu$V.

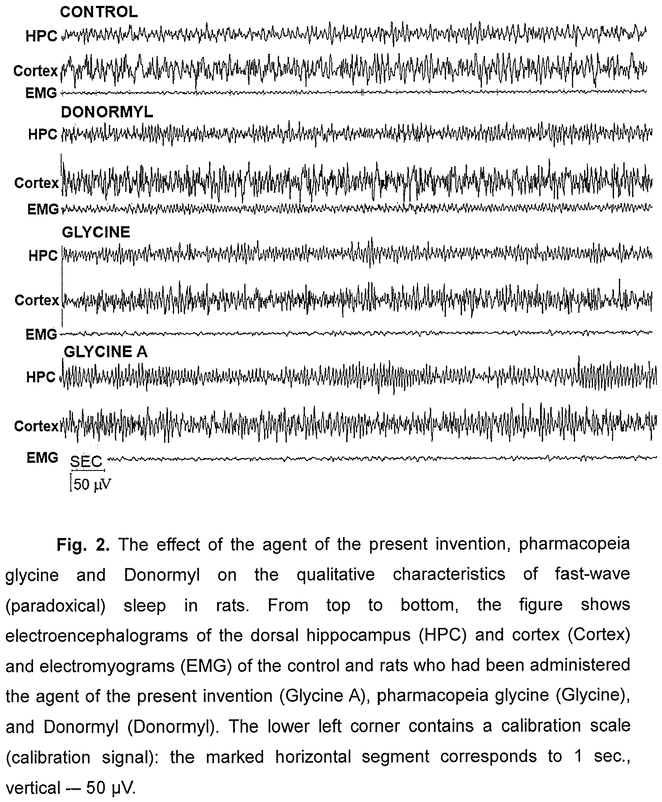

Fig.2. The effect of the agent of the present invention, pharmacopeia glycine and Donormyl on the qualitative characteristics of fast-wave (paradoxical) sleep in rats. From top to bottom, the figure shows electroencephalograms of the dorsal hippocampus (HPC) and cortex (Cortex) and electromyograms (EMG) of the control and rats who had been administered the agent of the present invention (Glycine A), pharmacopeia glycine (Glycine), and Donormyl (Donormyl). The lower left corner contains a calibration scale (calibration signal): the marked horizontal segment corresponds to 1 sec., vertical - 50 $\mu$V.

The present invention is illustrated by the following example:

Example. A study of the specific sedative-hypnotic activity of the agent of the present invention as compared to the pharmacopeia preparations glycine and Donormyl.

The study was conducted on 35 mature outbred male white rats weighing 230-250 g. The animals were received from the central nursery for laboratory animals "Stolbovaya", Moscow Oblast. The animals were kept in accordance with the principles of Good Laboratory Practice for nonclinical studies in the Russian Federation. The animals were kept in a vivarium at 20-22°C, with the light-dark cycle of 12 hours of light and 12 hours of darkness, in plastic cages T/4A, 580x375x200 mm in size, stainless steel cover, and dust-free litter covered with wood shavings, 8 rats per cage. The animals were allowed free access to food and water and were fed a full ration of extruded pelletized feed and drinking water. The experiments were conducted during the first half of the day.

The study of the sedative-hypnotic activity of the agent of the present invention was conducted in accordance with "Methodological Guidelines for Conducting a Study of Sedative-Hypnotic Activity of Pharmaceutical Substances" as set forth in "The Guidance Manual for Experimental (non-clinical) Studies of Novel Pharmaceutical Substances" [11]. The neurophysiological (electrophysiological) criteria of various stages of the sleep-wake cycle were analyzed.

Electrodes were surgically implanted into Nembutal-anesthetized rats (50 mg/kg, intramuscular) 5 - 7 days prior to the experiments on sleep monitoring. Long-term electrodes for registration of electrical activity were implanted in an encephalometer into the sensorimotor area of the cortex, dorsal hippocampus, and neck muscles. Dorsal hippocampus coordinates were calculated in accordance with the brain atlas of the rats [12]. Electrical activity of the brain and electromyograms of the neck muscles were recorded with nichrome wire electrodes with varnished insulation, 90 mcm in diameter for the hippocampus, and 120 mcm - for the sensorimotor area of the cortex and neck muscle implantation. The ends of the electrodes were soldered to silver-plated contacts 0.8 - 1 mm in diameter, which were affixed to the cranial bones with dental visfat-cement. During the experiment, the contacts were attached to lead wires connected to the electroencephalograph's control unit. The lead wires were connected to the control unit in such a special way (flexible and movable) as to record the electroencephalograms from freely moving animals over a long time period with no artefacts. Bioelectrical brain activity (electroencephalography, EEG) was recorded in the morning-daytime hours over at least 5 hrs. on a computerized electroencephalographic device, comprising software designed to analyze and evaluate the neurophysiological criteria of various sleep-wake cycle stages and statistical data processing.

The experiment was conducted as follows: 1st day - the animals were adapting to the experimental environment, 1-2 hrs.; 2nd day - background activity registration (5 hrs.), 3rd day - EEG recording over 5 hrs., 30 min. after the agent's administration. Sleep stages in the experiment were determined by measuring the electrical activity of the cerebral cortex and dorsal hippocampus and with an electrocardiogram. The software analyzing the sleep-wake cycles registered the following indicators: latent time from the beginning of registration to the appearance of the first episode of slow-wave (S) sleep in min. [LASS]; latent time from the beginning of registration to the appearance of the first episode of fast-wave (paradoxical, REM) sleep (F) in min. [LA F]; total number of slow-wave sleep episodes (S), [N(S)]; total number of fast-wave (paradoxical, REM) sleep episodes (F), [N(F)]; total number of episodes of wakefulness(W), [N(W)]; duration of all recordings in min. (total time of EEG recording no less than 5 hrs.) [T]; duration of all slow-wave sleep episodes (S) in min. [T(S)]; duration of all fast-wave (paradoxical, REM) sleep (F) episodes in min [T(F)]; duration of all wakefulness episodes (W) in min. [T(W)]; percentage of all slow-wave sleep episodes calculated by the ratio between the duration of slow-wave sleep and the duration of the total EEG recording period (S) [T(S)/T *100], [S%]; percentage of all fast-wave (paradoxical) sleep episodes calculated by the ratio between the duration of fast-wave sleep and the duration of the total EEG recording period, [T(F)/T*100], [F%]; percentage of all wakefulness episodes calculated by the ratio between the duration of wakefulness and the duration of the total EEG recording period, [T(W)/T*100], [W%]. Only the sleep-wake stages lasting at least 15 sec. were taken into consideration and calculated.

Statistical processing of the EEG results was conducted with the special "Brainsys Sleep" program. Statistical processing of the data was conducted with the "Statistica V. 6.0." program by one-way analysis of variance and the nonparametric test for variables (Mann-Whitney U-test).

Control rats' sleep, similar to the human sleep, is known to consist of distinctly alternating stages: 1- slow-wave sleep, 2- fast-wave sleep (fast, paradoxical, REM) and 3- wakefulness, and these sleep-wake cycles alternate several times during the entire sleep period.

Fig. 1 shows exemplary electroencephalograms of control rat #5, registered during different sleep-wake cycles.

The electrograms of the sensorimotor cortex and dorsal hippocampus (HPC) of the rats during wakefulness registered a dysthymic bioelectrical activity and the electromyograms (EMG) registered an intense muscular activity. Electrograms of the sensorimotor cortex and dorsal hippocampus (HPC) of the rats during the deep slow-wave sleep phase registered high-amplitude slow waves, and an electromyogram (EMG) showed a decreased muscular activity. The fast-wave sleep phase is preceded by an intermediate sub-stage of slow-wave sleep. The figure demonstrates that said sub-stage has a characteristic intense spindle-like activity. In the REM sleep stage, the sensorimotor cortex electrograms registered a distinct desynchronization of bioelectrical activity, and dorsal hippocampus (HPC) electrograms registered a well-defined hippocampal theta-rhythm with practically no muscular activity, which was confirmed by the sharply decreased amplitude in the neck muscle electrogram.

The study of the effect of the substances on the fast-wave and slow-wave sleep onset latency revealed that in the control animal group, the first onset of slow-wave sleep occurred, on the average, 17 min. after the start of EEG registration, and the first onset of fast-wave sleep occurred 69 min. after the start of EEG registration.

The agent of the present invention Almacin at a dose of 2 mg/kg demonstrated a statistically significant decrease of both slow-wave (26%) and fast-wave (21%) sleep onset latency as compared to control (Table 1).

Table 1. Effect of the agent of the present invention on the fast-wave and slow-wave sleep onset latency compared to the pharmacopeia glycine and Donormyl.

| Groups, Doses, Administration Mode, Number of Animals | Latency | |
|---|---|---|
| | Slow-wave sleep min. | Fast-wave sleep min. |
| Control n = 7 | 17.0±2.4 | 69.0±4.44 |
| Agent of the Present Invention 2 mg/kg, intraperitoneal, n = 7 | 12.6±3.4* | 55.0±3.51* |
| Pharmacopeia Glycine, 10 mg/kg, ip, n = 7 | 14.1±2.39 | 65.3±15.63 |
| Donormyl 15 mg/kg, ip, n = 7 | 19.3±7.41 | 96.3±17.55 |
| * - statistical significance compared to control at $P<0.05$ by the Mann-Whitney criterion | | |

Pharmacopeia glycine at a dose of 10 mg/kg did not show a statistically significant reduction in the fast-wave and slow-wave sleep onset latency compared to control. Donormyl at a dose of 15 mg/kg showed a trend toward some increase the fast-wave sleep onset latency compared to control and did not change the slow-wave sleep onset latency (Table 1).

The study of the effect of the substance of the present invention on the number of slow-wave and fast-wave sleep episodes demonstrated that compared to control, the substance of the present invention at a dose of 2 mg/kg showed a statistically significant increase (31%) in the number of fast-wave sleep episodes and reduction (27%) in the number of wakefulness episodes over a 5-hour registration period (Table 2).

Table 2. Effect of the agent of the present invention on the number of fast-wave and slow-wave sleep episodes and wakefulness compared to the pharmacopeia glycine and Donormyl.

| Groups, Doses, Administration Mode, Number of Animals | Number of Episodes | | |
|---|---|---|---|
| | Slow-Wave Sleep | Fast-Wave Sleep | Wakefulness |
| Control N = 7 | 39.3±1.46 | 14.7±1.59 | 38.7±1.46 |

(continued)

| Groups, Doses, Administration Mode, Number of Animals | Number of Episodes | | |
|---|---|---|---|
| | Slow-Wave Sleep | Fast-Wave Sleep | Wakefulness |
| Agent of the Present Invention 2 mg/kg, intraperitoneal, N = 7 | 33.9±1.81 | 19.4±1.96* | 28.4±2.53* |
| Pharmacopea Glycine, 10mg/kg, intraperitoneal, N = 7 | 32.3±2.50 | 15.2±2.55 | 29.0±2.09* |
| Donormyl 15 mg/kg, intraperitoneal, N = 7 | 31.7±2.43* | 12.0±1.98 | 30.2±2.95* |
| * - statistical significance compared to control at P<0.05 by the Mann-Whitney criterion | | | |

Pharmacopeia glycine at a dose of 10 mg/kg showed a statistically significant reduction (26%) in the number of wakefulness episodes, showed a trend towards a reduction in the number of slow-wave sleep episodes and had no effect on the fast-wave sleep episodes as compared to control. Donormyl at a dose of 15 mg/kg showed a statistically significant reduction (19%) in the slow-wave sleep episodes, 22% reduction in the number of wakefulness episodes, and had no effect on the number of fast-wave sleep episodes over a 5-hr. registration period (Table 2).

The study of the effect of the comparator substance on the total duration of slow-wave and fast-wave sleep and wakefulness demonstrated that the agent of the present invention at a dose of 2 mg/kg, over a 5-hr. registration period, showed a statistically significant increase (66%) in the total duration of fast-wave sleep and a 26% reduction of the duration of wakefulness (Table 3).

Table 3. Effect of the agent of the present invention on the total duration of slow-wave sleep, fast-wave sleep and wakefulness compared to the pharmacopeia glycine and Donormyl.

| Groups, Doses, Administration Mode, Number of Animals | Duration, min | | | |
|---|---|---|---|---|
| | total EEG recording period | Slow-Wave Sleep | Fast-Wave Sleep | Wakefulness |
| Control, n = 7 | 335.8±6.18 | 206.3±7.47 | 16.6±2.78 | 111.5±9.58 |
| Agent of the Present Invention 2 mg/kg, intraperitoneal, N = 7 | 315.8±8.53 | 205.0±18.17 | 27.6±3.93* | 83.5±10.69* |
| Pharmacopea Glycine, 10mg/kg, intraperitoneal, N = 7 | 322.6±4.31 | 210.4±5.73 | 19.8±3.11 | 92.5±5.54 |
| Donormyl 15 mg/kg, intraperitoneal, N = 7 | 321.9±6.16 | 216.6±6.83 | 20.1 ±3.63 | 85.0±11.87* |
| * - statistical significance compared to control at P<0.05 by the Mann-Whitney criterion | | | | |

Pharmacopeia glycine at a dose of 10 mg/kg showed a trend towards an increase in the fast-wave sleep duration and decreased the duration of wakefulness compared to control. Over a 5-hr. registration period, Donormyl at a dose of 15 mg/kg showed a statistically significant reduction (24%) in the duration of wakefulness, and showed a trend towards an increase in the duration of slow-wave and fast-wave sleep compared to control (Table 3).

Percentage of the duration of slow-wave and fast-wave sleep episodes and wakefulness were calculated by the ratio between the duration of the sleep phase to the duration of the total EEG recording period according to the following formula:

$$\frac{\text{Duration of sleep phase}}{\text{Duration of the whole EEG}} \times 100\% \ .$$

The substance of the present invention at a dose of 2 mg/kg, over a 5-hr. registration period showed a statistically significant increase in the percentage of fast-wave sleep and a decrease in the percentage of wakefulness compared to control (Table 4).

Pharmacopeia glycine at a dose of 10 mg/kg showed a statistically significant reduction in the percentage of the duration of wakefulness and showed a trend towards an increase in the duration of fast-wave sleep as compared to control. Over a 5-hr. registration period Donormyl at a dose of 15 mg/kg showed a statistically significant reduction in the percentage of wakefulness and showed a trend towards an increase in the percentage of slow-wave and fast wave sleep as compared to control (Table 4).

| **Table 4.** Effect of the agent of the present invention on the <u>percentage</u> of the duration of all slow-wave sleep, fast-wave sleep and wakefulness episodes compared to glycine and Donormyl.**Group s, Doses, Administration Mode, Number of Animals** | **Ratio between the duration of slow-wave sleep and the duration of total recording period, %** | **Ratio between the duration of fast-wave sleep and the duration of total recording period, %** | **Ratio between the duration of wakefulness and the duration of total recording period, %** |
|---|---|---|---|
| Control, n = 7 | 61.6±2.49 | 4.9±0.78 | 33.2±2.73 |
| Agent of the Present Invention 2 mg/kg, intraperitoneal, N = 7 | 64.4±4.62 | 8.8±1.25* | 26.8±3.80* |
| Pharmacopea Glycine, 10mg/kg, intraperitoneal, N = 7 | 65.2±1.52 | 6.1±0.94 | 28.7±1.73* |
| Donormyl 15 mg/kg, intraperitoneal, N = 7 | 67.5±3.62# | 6.3±1.16 | 26.1±4.39* |
| * - statistical significance compared to control at P<0.05 by the Mann-Whitney criterion | | | |

The agent of the present invention has a significant impact on the quality of fast-wave (paradoxical, REM) sleep. Said agent was shown to enhance the theta-rhythm synchronization and modulation in the electrograms of the sensorimotor cortex and dorsal hippocampus in the fast-wave (paradoxical) sleep phase. Said qualitative changes in the electroencephalograms are shown in Fig. 2

The ability of the agent of the present invention to enhance the theta-rhythm synchronization and modulation in the electrograms of the sensorimotor cortex and dorsal hippocampus in the fast-wave (paradoxical) sleep phase indicate the normalizing effect of the substance on cortical-diencephalic relationships involved in the electrophysiological and neurochemical balance regulations in the central nervous system during sleep

As opposed to the claimed agent, pharmacopeia glycine and Donormyl did not enhance the theta-rhythm synchronization and modulation in the electrograms of the sensorimotor cortex and dorsal hippocampus in the fast-wave (paradoxical) sleep phase (Fig. 2).

The study results obtained in the examples indicate that the agent of the present invention (at a 2 mg/kg dose) exhibits a pronounced sedative-hypnotic effect exceeding the sedative-hypnotic effect of the pharmacopeia glycine (at a 1- mg/kg dose) and the comparator agent Donormyl (at a 15 mg/kg dose).

## LITERATURE

1. N.B. Leonidov, R.Yu. Yakovlev, G.V. Lisichkin. Sedative agent and method for the preparation thereof. Pat. RF 2506075,2013.

2. Somnology and medicine of sleep. Selected lectures/ edited by Ya. I. Levin, M.G. Poluektova. M.: Medforum, 2013. pp. 208-214.

3. M.D. Mashkovsky. Medicinal agents. 16th edition, revised, corrected, expanded -M.: Novaya Volna: Publisher: Umerenkov, 2012. pp. 34-50.

4. A.Yu. Bespalov, E.E. Zvartau. Neuropsychopharmacology of NMDA-receptor antagonists. SPb.: Nevsky Dialect, 2000. 297 p.

5. I.A. Komisarova. Ya. R. Narcissov. Molecular mechanisms of the effect of the drug "Glycine" // Terra medica. 2001. #1. pp. 23-25.

6. Brooks PL, Peever JH. Impaired GABA and glycine transmission triggers cardinal features of rapid eye movement sleep behavior disorder in mice // J Neurosci. 2011. V.31. № 19. P.7111-21).

7. Hondo M, Furutani N, Yamasaki M, Watanabe M, Sakurai T. Orexin neurons receive glycinergic innervations // PLoS One. 2011. V. 6. № 9, e25076.

8. N.B. Leonidov, R.Yu. Yakovlev, A.S. Solomatin, G.V. Lisichkin. Antidepressant and method for the preparation thereof. Pat. RF 2519759, 2013.

9. N.B. Leonidov, R.Yu. Yakovlev, G.V. Lisichkin. Antipsychotic agent and method for the preparation thereof. Pat. RF 2519761, 2013.

10. N.B. Leonidov, R.Yu. Yakovlev, I.I. Kulakova, G.V. Lisichkin. Anxiolytic agent and method for the preparation thereof. Pat. RF 2519755, 2013.

11. T.A. Voronina, L.N. Nerobokova. Methodology guidelines for the study of sedative-hypnotic activity of pharmaceutical substances// Handbook for the experimental (non-clinical) study of novel pharmaceutical substances / edited R.U. Khabriev (Federal Service of Healthcare and Social Development Control, FSI Scientific Center for Evaluation of Medical Products) M.: Medicina, 2005. pp. 263-276.

12. Ya. Buresh, O. Bureshova, P. Huston. Methodology and basic experiments in the study of the brain and behavior. M.: Vyshaya Shkola 1991, 399 p.

**Claims**

1.  A pharmaceutical composition comprising a conjugate of glycine immobilized on detonation nanodiamond particles, 2-10 nm in size, wherein the content of glycine is up to 21 +/-3 wt% of glycine, for use in the treatment or prevention of a sleep disorder.

2.  The pharmaceutical composition for use according to claim 1, wherein the sleep disorder is insomnia.

3.  The pharmaceutical composition for use according to claim 1 or claim 2, wherein said composition reduces the onset latency of first episodes and the total number of all episodes of slow-wave and fast-wave sleep as well as the duration and percentage of wakefulness, while increasing the duration and percentage of fastwave sleep.

**Patentansprüche**

1.  Pharmazeutische Zusammensetzung mit einem Konjugat aus Glyzin, das durch die Detonation Nanodiamant-Partikel bewegengunfähig macht, wobei die Partikel eine Größe von 2-10nm aufweisen und der Glyzingehalt bis zu 21 ±3 Gewichts-% beträgt, für Zwecke der Behandlung oder Prävention der Schlafkrankheit.

2.  Pharmazeutische Zusammensetzung für die Zwecke nach Anspruch 1,
    **dadurch gekennzeichnet,**
    **dass** die Schlafkrankheit Insomnie ist.

3.  Pharmazeutische Zusammensetzung für die Zwecke nach Anspruch 1 oder 2,
    **dadurch gekennzeichnet,**
    **dass** die Zusammenstellung die Anfangswartezeit der ersten Episoden und die Gesamtanzahl aller Episoden des Langwellen- und Schnellwellenschlafs sowie die Dauer und den Prozentsatz des Wachseins vermindert, während sie die Dauer und den Prozentsatz des Schnellwellenschlafs erhöht.

**Revendications**

1.  Composition pharmaceutique avec un conjugué de glycine qui rend invalide grâce à la détonation de particules de nano-diamants de sorte que ces particules présentent une taille de 2-10nm et que le poids du contenu de la glycine s'élève à 21 ±3 -% utilisée dans le traitement ou le cas échéant dans la prévention de la maladie du sommeil.

2. Composition pharmaceutique aux fins indiquées selon la revendication 1,
est **caractérisée** de sorte que
la maladie du sommeil est une insomnie.

3. Composition pharmaceutique aux fins indiquées selon la revendication 1 ou 2,
est **caractérisée** de sorte que
la composition réduit le temps d'attente initial des premières épisodes et le nombre total de toutes les épisodes du sommeil à ondes longues et à ondes rapides ainsi que la durée et le pourcentage de la vigilance, tandis qu'elle augmente la durée et le pourcentage du sommeil à ondes rapides.

**Fig. 1.** Electroencephalograms of control rat #5 representing different stages (phases) of the sleep-wake cycle. From top to bottom, the figure shows electroencephalograms of the dorsal hippocampus (HPC) and cortex and electromyograms (EMG) of the wakefulness, slow-wave and fast-wave sleep phases. The lower right corner contains a calibration scale (calibration signal): the marked horizontal segment corresponds to 1 sec., vertical — 50 μV.

**Fig. 2.** The effect of the agent of the present invention, pharmacopeia glycine and Donormyl on the qualitative characteristics of fast-wave (paradoxical) sleep in rats. From top to bottom, the figure shows electroencephalograms of the dorsal hippocampus (HPC) and cortex (Cortex) and electromyograms (EMG) of the control and rats who had been administered the agent of the present invention (Glycine A), pharmacopeia glycine (Glycine), and Donormyl (Donormyl). The lower left corner contains a calibration scale (calibration signal): the marked horizontal segment corresponds to 1 sec., vertical — 50 μV.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **N.B. LEONIDOV ; R.YU. YAKOVLEV ; G.V. LISICHKIN.** *Sedative agent and method for the preparation thereof,* 2013
- Somnology and medicine of sleep. Selected lectures. Medforum, 2013, 208-214
- **M.D. MASHKOVSKY.** Medicinal agents. Umerenkov, 2012, 34-50
- **A.YU. BESPALOV ; E.E. ZVARTAU.** Neuropsychopharmacology of NMDA-receptor antagonists. Nevsky Dialect, 2000, 297
- **I.A. KOMISAROVA ; YA. R. NARCISSOV.** Molecular mechanisms of the effect of the drug ''Glycine. *Terra medica,* 2001, vol. 1, 23-25
- **BROOKS PL ; PEEVER JH.** Impaired GABA and glycine transmission triggers cardinal features of rapid eye movement sleep behavior disorder in mice. *J Neurosci.,* 2011, vol. 31 (19), 7111-21
- **HONDO M ; FURUTANI N ; YAMASAKI M ; WATANABE M ; SAKURAI T.** Orexin neurons receive glycinergic innervations. *PLoS One,* 2011, vol. 6 (9), e25076
- **N.B. LEONIDOV ; R.YU. YAKOVLEV ; A.S. SOLOMATIN ; G.V. LISICHKIN.** *Antidepressant and method for the preparation thereof,* 2013
- **N.B. LEONIDOV ; R.YU. YAKOVLEV ; G.V. LISICHKIN.** *Antipsychotic agent and method for the preparation thereof,* 2013
- **N.B. LEONIDOV ; R.YU. YAKOVLEV ; I.I. KULAKOVA ; G.V. LISICHKIN.** *Anxiolytic agent and method for the preparation thereof,* 2013
- Methodology guidelines for the study of sedative-hypnotic activity of pharmaceutical substances. **T.A. VORONINA ; L.N. NEROBOKOVA.** Handbook for the experimental (non-clinical) study of novel pharmaceutical substances. Federal Service of Healthcare and Social Development Control, FSI Scientific Center for Evaluation of Medical Products, 2005, 263-276
- **YA. BURESH ; O. BURESHOVA ; P. HUSTON.** *Methodology and basic experiments in the study of the brain and behavior,* 1991, 399